# EUROPEAN PATENT APPLICATION

(11) **EP 1 862 132 A1**
(43) Date of publication of application: **05.12.2007**
(21) Application number: 06715022.7
(22) Date of filing: 01.03.2006
(51) Int. Cl.: A61B 17/221, A61B 17/32, A61B 1/00

(54) **GUIDE WIRE-TYPE TREATMENT**

(30) Priority: 24.03.2005 JP 2005085750
(71) Applicant: Olympus Corporation, Tokyo 151-0072 (JP)
(72) Inventor: YANUMA, Yutaka, Kunitachi-shi, Tokyo 1860004 (JP)
(74) Representative: von Hellfeld, Axel
(86) International application number: PCT/JP2006/303906
(87) International publication number: WO 2006/100882

(57) **Abstract**

A guide wire treatment tool includes an operation wire having a treatment part on the head thereof; and a sheath through which the operation wire is slidably inserted. A head chip having a through-hole, through which a guide wire is inserted, is provided on a head part of the treatment part, and the treatment part and the head chip are coupled via a coupling part so as to be relatively rotatable around an axis which extends along the length of the operation wire.

## Description

### TECHNICAL FIELD

The present invention relates to a guide wire (type) treatment tool used together with an endoscope, for performing various kinds of treatments.

Priority is claimed on Japanese Patent Application No. 2005-085750, filed March 24, 2005, the content of which is incorporated herein by reference.

### BACKGROUND ART

Recently, in medical fields or the like, a treatment tool for an endoscope has been used for performing various kinds of treatments while monitoring an image captured by the endoscope. A guide wire treatment tool is a known example of such a treatment tool, which includes a sheath having two lumens, and an operation wire having a brush at the head thereof, wherein the operation wire is inserted into one of the lumens, and a guide wire for guiding the operation wire is inserted into the other lumen (see, for example, Patent Document 1).

However, in the above structure, the two lumens are arranged parallel to each other inside the sheath, and thus the sheath needs to be broad and hard. Therefore, it is difficult to make such a sheath pass along a curved portion of a narrowed part, papilla, bile duct, pancreatic duct, or the like.

Therefore, a tool is also known in which a head chip having a through-hole is provided on the head of a treatment part, and a guide wire is inserted through the through-hole (see, for example, Patent Document 2). In accordance with this structure, the operation wire can be guided without inserting the guide wire through the sheath, so that the sheath can be thin and soft.

In order to collect cells in a coelom by using a brush, generally, the brush is guided and positioned at an affected part (e.g., a narrowed part), and the brush is moved forward and backward by moving the operation wire forward and backward at that position, so as to scrape off and collect cells. However, when moving the brush forward and backward after guiding the brush to a gap of the narrowed part, the brush may fall off. In such a case, the brush needs to be sent to the gap of the narrowed part again, which is inconvenient. Therefore, in some situations, the brush is not moved forward and backward, and cells are scraped off and collected by rotating the brush around the axis along the length of the operation wire.
Patent Document 1: U.S. Patent No. 5,427,115
Patent Document 2: Japanese Unexamined Patent Application, First Publication No. 2004-249093

However, in the above structure having the head chip, when the brush is rotated around the above axis, the guide wire is also rotated via the head chip around the axis. Accordingly, the operation wire and the guide wire catch each other, and thus it is difficult to satisfactorily perform an operation.

### DISCLOSURE OF INVENTION

In light of the above circumstances, an object of the present invention is to provide a guide wire (type) treatment tool by which

Therefore, the present invention provides a guide wire treatment tool comprising:
an operation wire having a treatment part on the head thereof; and
a sheath through which the operation wire is slidably inserted,
wherein a head chip having a through-hole, through which a guide wire is inserted, is provided on a head part of the treatment part, and the treatment part and the head chip are coupled via a coupling part so as to be relatively rotatable around an axis which extends along the length of the operation wire.

In the above guide wire treatment tool, when the operation wire is rotated around the above axis, the treatment part rotates. In this process, it is possible to rotate only the operation wire and the treatment part without moving the head chip, by means of the coupling part. Therefore, when rotating the operation wire and the treatment part, it is possible to prevent the guide wire from interfering with the operation wire.

In a preferable example:
in the head chip, a recessed part is provided on an end thereof, wherein said end faces the treatment part;
the coupling part includes:
   a coupling member, which is provided on the head part of the treatment part, and
   a flange part, which extends inwardly in a radial direction and has an opening whose diameter is smaller than the outer diameter of the coupling member; and
the head part of the treatment part is rotatably held in the recessed part via the coupling member which is engaged with the flange part.

In this structure, when pulling the operation wire, the coupling part is engaged with the flange part, thereby preventing the operation wire from being disconnected. In addition, when rotating the operation wire, the coupling part also rotates in the recessed part via the treatment part, together with the operation wire.

Therefore, the operation wire and the head chip can be coupled with each other, and the operation wire can be reliably rotated, by employing a simple structure.

A spacer member may be provided, with which at least a part of a space between the inner face of the sheath and the outer face of the head part in the treatment part is filled when the head chip is arranged on the head of the sheath.

In this structure, when the head chip is arranged on the head of the sheath, at least a part of a space between the inner face of the sheath and the outer face of the head part in the treatment part is filled with the spacer member. Therefore, if the head part of the treatment part tries to move in the sheath toward a direction which intersects the above axis, movement of the head part is restricted by the spacer member, thereby also restricting movement of the head chip in the same direction.

Accordingly, when the head chip is disposed on the head of the sheath, it is possible to prevent an offset between the head chip and the sheath. Therefore, it is possible to smoothly insert and move the sheath in a coelom.

In accordance with the present invention, even when rotating the operation wire and the treatment part, it is possible to prevent the guide wire from interfering with the operation wire. Therefore, a thin and soft sheath can be retained, and the treatment part can be quickly and appropriately operated with regard to various situations.

### BRIEF DESCRIPTION OF THE DRAWINGS

Fig. 1 is a diagram for showing a first embodiment of the guide wire treatment tool in accordance with the present invention, and for explaining a state in which various treatments are performed together with an endoscope.
Fig. 2 is an enlarged side view of the guide wire treatment tool in Fig. 1.
Fig. 3 is a sectional view showing a head part of the treatment tool in Fig. 1.
Fig. 4A is a diagram showing a treatment for collecting cells in a narrowed part, and explaining a state in which an insertion part of the endoscope is disposed in the vicinity of a papilla, and an imaging catheter is extended.
Fig. 4B is a diagram also showing the treatment for collecting cells in the narrowed part, and explaining a state in which a guide wire, which extends from the head of the imaging catheter, passes through the narrowed part and reaches ahead thereof.
Fig. 4C is a diagram also showing the treatment for collecting cells in the narrowed part, and explaining a state in which only the guide wire passes through the narrowed part and is positioned ahead thereof
Fig. 5A is a diagram showing the treatment for collecting cells in the narrowed part, and explaining a state in which a sheath is inserted and a head chip is disposed in the narrowed part.
Fig. 5B is a diagram also showing the treatment for collecting cells in the narrowed part, and explaining a state in which a brush is arranged in the narrowed part.
Fig. 6 is a side sectional view showing a second embodiment of the guide wire treatment tool in accordance with the present invention.
Fig. 7 is a diagram for showing and explaining a state in which the head chip in Fig. 6 is disposed on the head of the sheath, so as to insert the sheath into a bile duct.
Fig. 8 is a diagram for showing and explaining a state in which an offset occurs between the sheath and the head chip when providing no spacer member in the guide wire treatment tool of Fig. 6.
Fig. 9 is a side sectional view showing a third embodiment of the guide wire treatment tool in accordance with the present invention.
Fig. 10 is a side sectional view showing a fourth embodiment of the guide wire treatment tool in accordance with the present invention.
Fig. 11 is a front view of the head chip shown in Fig. 10.
Fig. 12 is a side sectional view showing a fifth embodiment of the guide wire treatment tool in accordance with the present invention.
Fig. 13 is a side sectional view showing a state in which the cup pieces in Fig. 12 are opened.
Fig. 14 is a side sectional view showing a sixth embodiment of the guide wire treatment tool in accordance with the present invention.

### BEST MODE FOR CARRYING OUT THE INVENTION

First embodiment

A guide wire treatment tool as a first embodiment of the present invention will be explained with reference to the drawings.

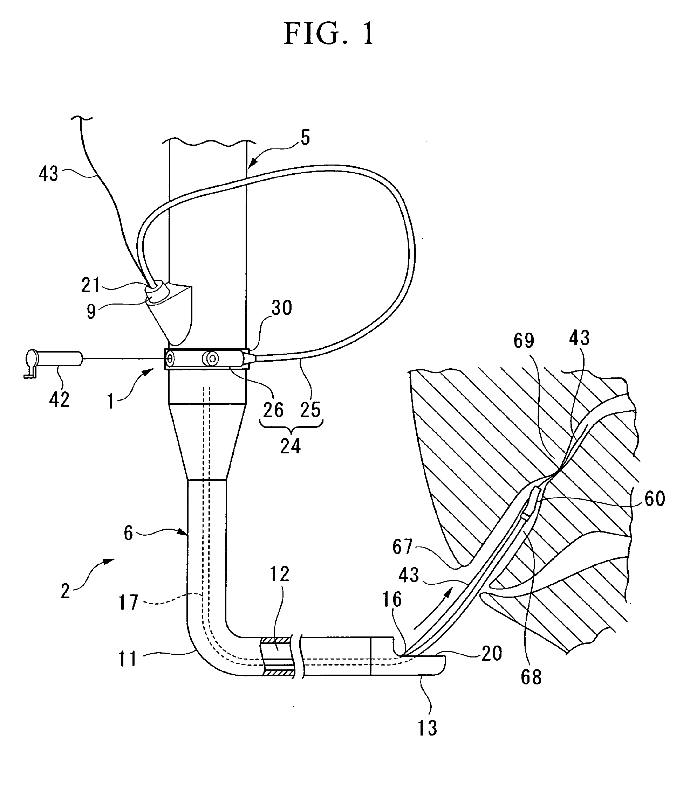

As shown in Fig. 1, a guide wire (type) treatment tool 1 of the present embodiment is used together with an endoscope 2, in order to treat an affected area.

First, the endoscope 2, used together with the guide wire treatment tool 1, will be explained.

The endoscope 2 has, as main structural elements, an endoscope operating part 5, handled by an operator so as to perform various operations, and an endoscope insertion part 6 inserted in a coelom such as a duodenum. That is, in the endoscope 2, the endoscope operating part 5 is provided so as to couple with an end (closer to the operator's hand) of the endoscope insertion part 6, which has an elongated hollow form.

In addition, the endoscope 2 may form an endoscope system when it is appropriately combined with another external device (not shown) depending on the purpose, which may be a light source, an image processing apparatus, a monitor, a key board for data input, a suction pump, a water supply bottle, or the like. Generally, such an external device is installed on a shelf having a carrier. Among the above external devices, the light source or the image processing apparatus is coupled with the endoscope operating part 5 via a universal cord (not shown).

The endoscope operating part 5 has an operation lever and operation buttons (not shown) for performing various treatment operations, and a forceps plug 9 for inserting the guide wire treatment tool 1 is provided on the head of the endoscope operating part 5. The forceps plug 9 has a forceps plug opening 21 for inserting the guide wire treatment tool 1, and the forceps plug opening 21 is in communication with a tube-form channel 17, which is an insertion passage for the guide wire treatment tool 1.

In addition, the endoscope insertion part 6 includes a flexible tube part 11, whose base end is coupled with the endoscope operating part 5, and which is flexible and has a long form; a curved part 12, provided on the head of the flexible tube part 11, for curving the endoscope insertion part 6; and a head part 13 provided on the head of the curved part 12.

On the outer periphery of the head part 13, a part of the side face is removed so as to form a recessed cut part 20, and a channel exit opening 16 is formed on a side-face part of the cut part 20. The channel exit opening 16 is in communication with the forceps plug opening 21 via the channel 17. In addition, on a side of the channel exit opening 16, an objective (lens) of an observation optical system and an illumination lens of an illumination optical system (both not shown) are arranged side by side. Furthermore, on the rear wall face of the cut part 20, an air/water supply nozzle (not shown) is provided for cleaning the objective and the illumination lenses.

As specific examples, the operation lever and the operation buttons may be a curved-part operation lever for moving the curved part 12 upward, downward, rightward, or leftward, water/air supply buttons for selectively jetting a gas or a liquid to the air/water supply nozzle at the head part 13, or a button for selectively applying an absorptive force to the channel exit opening 16 so as to collect a mucus or the like in the coelom.

Next, the guide wire treatment tool 1 with respect to the present invention will be explained.

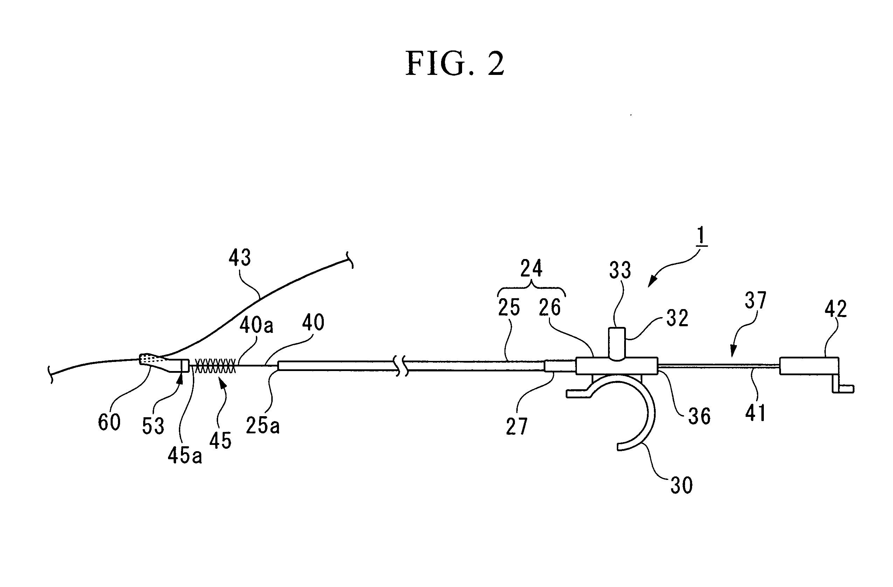

As shown in Fig. 2, the guide wire treatment tool 1 has a tube-form sheath part 24, and a tool main body 37 for performing various kinds of treatments.

The sheath part 24 includes a flexible sheath 25 (i.e., a sheath) inserted into a coelom, and a base-end support part 26 for supporting the flexible sheath 25. The flexible sheath 25 and the base-end support part 26 are coupled with each other via a breakage preventing part 27.

At the base-end support part 26, a water supply cock 32 is provided, which is in communication with the inside of the flexible sheath 25, and a connection opening 33 is provided at the water supply cock 32. A syringe (not shown) is connected to the connection opening 33, so that water can be supplied to the flexible sheath 25 via the water supply cock 32. In addition, at the base end of the base-end support part 26, an entrance opening 36 is formed, which is in communication with the flexible sheath 25. An elastic hook member 30 is provided at the base-end support part 26.

The hook member 30 is provided for attaching the guide wire treatment tool 1 to the endoscope 2 shown in Fig. 1. That is, when the hook member 30 is pushed against the endoscope operating part 5, it is elastically deformed and fits to the endoscope operating part 5, so that the sheath part 24 is detachably attached to the endoscope operating part 5.

The tool main body 37 includes an operation wire 40 and a hard pipe 41, which are coupled coaxially.

At the base end of the pipe 41, a handle part 42 is provided. When rotating the handle part 42 around the axis of the operation wire 40 and the hard pipe 41, the operation wire 40 is rotated via the pipe 41.

At the head 40a of the operation wire 40, a brush 45 (i.e., a treatment part) made of nylon is provided. In addition, to the head part 45a of the brush 45, a head chip 60 for arranging the brush 45 at a specific position is coupled via a coupling part 53.

The head chip 60 may be made of metal such as stainless steel. However, when using such a hard material, a guide wire 43 may be damaged due to an insufficient edge treatment of the chip. Therefore, a material softer than metal is preferable. For example, the following materials may be used: fluorocarbon polymers (or fluororesin, e.g., polytetrafluoroethylene or FEP), various kinds of rubber (e.g., silicone rubber), various kinds of elastomer (e.g., polyamide elastomer or polyester elastomer ("Hytrel", a registered trademark of DuPont), and other plastics (e.g., polyamide, ethylene-vinyl acetate copolymer, polyethylene, polypropylene, poly (etheretherketone) (PEEK), polycarbonate, polysulfone, or acrylonitrile-styrene-butadiene). Additionally, in order to pass through a curved portion of the papilla, bile duct, pancreatic duct, or the like, a soft material which can follow the curved part is preferable. For that purpose, elastomer is more preferable. However, if it is too soft, a though-hole 55 (explained later) tends to break. In addition, when using a material having inferior smoothness, the head chip cannot smoothly move along the guide wire 43. Therefore, a more preferable material can be obtained by combining some of the above materials (e.g., a combination of a polyamide elastomer and polyurethane).

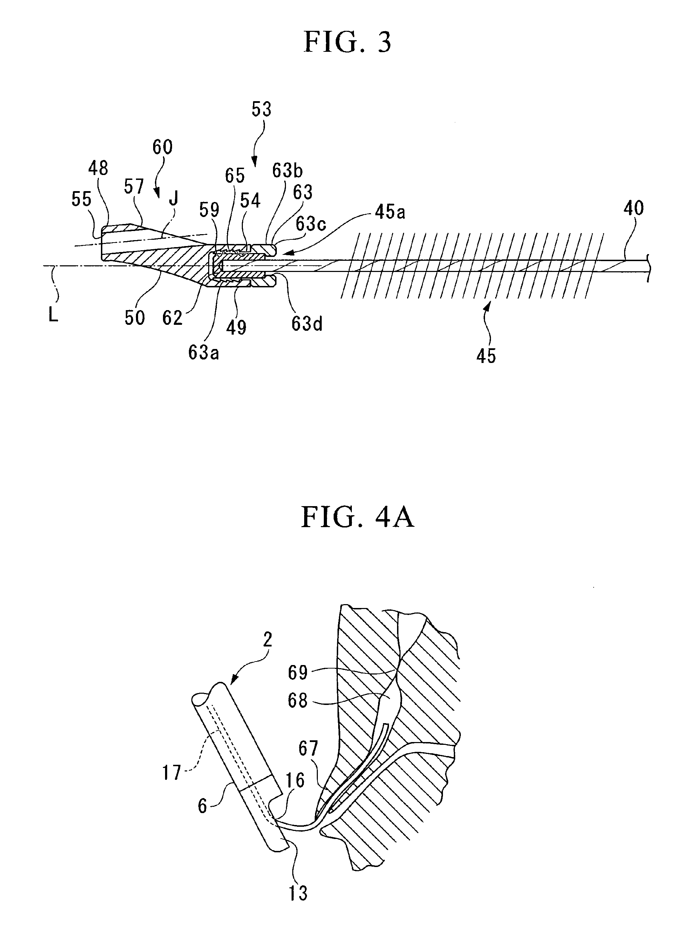

As shown in Fig. 3, the head chip 60 has (i) a chip head part 48, in which the through-hole 55 is formed, through which the guide wire 43 passes, and (ii) a chip rear-end part 49, which extends along the axis L of the operation wire 40. The head chip 60 is formed by integrally coupling the chip head part 48 and the chip rear-end part 49 via an offset part 50. That is, the central axis of the chip rear-end part 49, which coincides with the axis L of the operation wire 40, is offset from the central axis J of the through-hole 55 by means of the offset part 50.

The chip head part 48 is inclined so that the head having the through-hole 55 gradually approaches the axis L. This arrangement is employed for easily sending the head chip 60 even to a narrowed passage in a coelom, and also for easily and accurately sending the brush 45 toward the intersection of the axis L and the central axis J by arranging the guide wire 43 (which passes through the through-hole 55) on the axis L. At the rear end of the chip head part 48, a rear-end cut part 57 is formed by inclinedly cutting the relevant rear end toward the front end. The rear-end cut part 57 is provided for preventing the head chip 60 from being caught when it is pulled in a coelom so as to be returned, and thus for easily returning the head chip 60.

The chip rear-end part 49 has a recessed part 54 extending along the axis L, in which a cylindrical fixed part 63 is provided. Along the inner wall of the recessed part 54, fittable inclined steps 59 are arranged along the axis L.

The cylindrical fixed part 63 has a main cylindrical part 63 a, and a larger-diameter part 63b having an outer diameter larger than that of the main cylindrical part 63a. On the outer wall of the main cylindrical part 63a, fittable inclined steps 65 are arranged along the axis L, which are engaged with the fittable inclined steps 59. At the rear end of the larger-diameter part 63b, an inward flange part 63c is formed, which extends inward along the radial direction of the recessed part 54. Additionally, in the recessed part 54, a coupling member 62 is provided, which may be made of metal. The coupling member 62 extends along the axis L, and has a cylindrical form having a bottom. Into the opening of this cylinder, the head part 45a of the brush 45 is inserted and fixed. The coupling member 62, which is connected to the operation wire 40 via the brush 45, does not become disconnected from the recessed part 54 by means of the inward flange part 63c. That is, the outer diameter of the coupling member 62 is larger than an opening 63d.

The recessed part 54, the cylindrical fixed part 63, the coupling member 62, and the like form the coupling part 53, and the assembly thereof may be performed as explained below.

First, the coupling member 62 is fastened to the head part 45a of the brush 4. Then the rear end of the operation wire 40 is inserted into the opening of the cylindrical fixed part 63, and the cylindrical fixed part 63 is moved toward the head part 45a. The cylindrical fixed part 63 is moved over the brush 45, and disposed at the head part 45a. The cylindrical fixed part 63 is then inserted into the recessed part 54 while the coupling member 62 is disposed in the cylindrical fixed part 63. Accordingly, the fittable inclined steps 65 are engaged with the fittable inclined steps 59, and the cylindrical fixed part 63 is fixed in the recessed part 54, so that the coupling member 62 is rotatably supported in the recessed part 54.

In the above structure, when the handle part 42 shown in Fig. 2 is pulled, the brush 45 is contained in the flexible sheath 25 via the pipe 41 and the operation wire 40 and the head chip 60 moves toward the head 25a of the flexible sheath 25. In contrast, when pushing the handle part 42 toward the base-end support part 26, the brush 45 appears from the head 25a of the flexible sheath 25. In addition, when rotating the handle part 42 along the axis L, the brush 45 is rotated via the pipe 41 and the operation wire 40.

Next, the method of using the guide wire treatment tool 1 having the above structure in the present embodiment will be explained. In the present embodiment, as shown in Figs. 4A to 5B, on the middle of a bile duct 68, a narrowed part 69 is formed, which blocks the bile duct 68. An example of treatment for collecting cells of the narrowed part 69 will be explained as an example.

First, the endoscope operating part 5 is handled so as to insert the endoscope insertion part 6 into the duodenum. Next, as shown in Fig. 4A, an imaging catheter is inserted into the forceps plug opening 21, and is protruded via the channel 17 from the channel exit opening 16 of the head part 13, thereby inserting the imaging catheter into the bile duct 68.

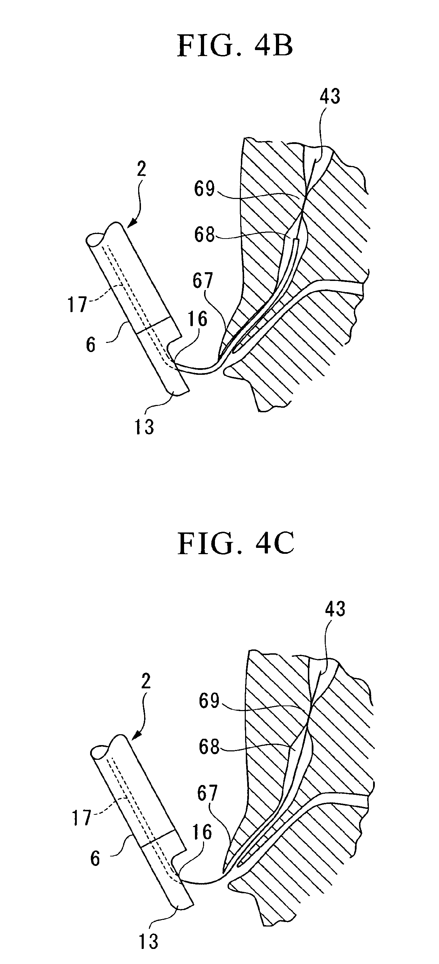

After the imaging catheter is inserted into the bile duct 68, the guide wire 43 is inserted into the inlet (closer to the operator) of the opening in the imaging catheter (see Fig. 4B), so that the head of the guide wire 43 is protruded from the head of the imaging catheter. The guide wire 43 is further protruded so that it protrudes further than the narrowed part 69. After the guide wire 43 reaches a position ahead of the narrowed part 69, only the imaging catheter is pulled out from the bile duct 68 and the channel 17 without moving the guide wire 43, so that only the guide wire 43 is positioned ahead of the narrowed part 69 (see Fig. 4C).

Next, the guide wire treatment tool 1 is inserted into the narrowed part 69 by the means of the guide wire 43 inserted ahead of the narrowed part 69. In this process, first, the handle part 42 is pulled so that the brush 45 is contained in the flexible sheath 25, so as to position the head chip 60 close to the head 25a of the flexible sheath 25. In the next step, the guide wire treatment tool 1 is attached to the endoscope operating part 5 via the hook member 30 (see Fig. 1). Then, the rear end of the guide wire 43, which extends from the forceps plug opening 21, is inserted through the through-hole 55, that is, from the head to the rear end of the chip head part 48 shown in Fig. 3.

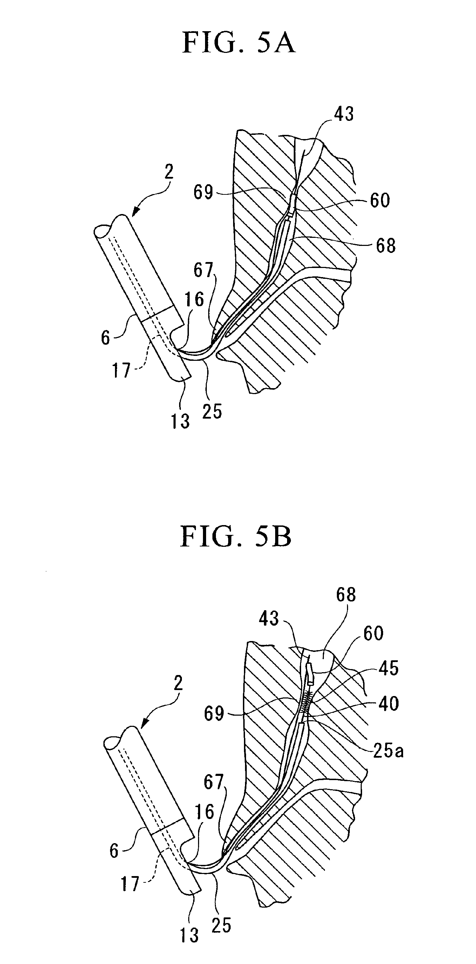

The flexible sheath 25 is inserted into the forceps plug opening 21 while the guide wire 43 is inserted through the through-hole 55. Thus, only the guide wire treatment tool 1 is moved forward through the channel 17 without moving the guide wire 43, thereby protruding the guide wire treatment tool 1 from the channel exit opening 16 of the head part 13. As shown in Fig. 5A, the guide wire treatment tool 1 is inserted to the narrowed part 69 in the bile duct 68. Then, the handle part 42 is pushed so that in the narrowed part 69, the brush 45 is protruded from the sheath 25 (see Fig. 5B).

In this state, if the brush 45 is moved forward and backward, it tends to become disconnected from the narrowed part 69.

Therefore, the operation wire 40 is rotated by operating the handle part 42, so that the brush 45 rotates in the same direction while remaining in the narrowed part 69. Accordingly, the inner wall of the narrowed part 69 is scratched by the brush 45, and cells are removed and adhered to the brush 45. In this state, the operation wire 40 is pulled so that the brush 45 is contained in the flexible sheath 25. Then, the flexible sheath 25 and the guide wire 43 are pulled out from the relevant papilla 67, thereby collecting the cells of the narrowed part 69.

If the operation wire 40 is rotated in this state, the guide wire 43 inserted through the through-hole 55 may be rotated in the same direction via the head chip 60. In the guide wire treatment tool 1 of the present embodiment, such a rotation of the guide wire 43 is prevented as explained below.

As shown in Fig. 3, when the operation wire 40 is rotated, the coupling member 62 attached to the head part 45a of the brush 45 rotates in the recessed part 54. Therefore, only the operation wire 40 relatively rotates while the head chip 60 in the narrowed part 69 is not moved. Accordingly, the guide wire 43 still remains at the specific position without being rotated.

In accordance with the guide wire treatment tool 1, only the operation wire 40 can be rotated without moving the head chip 60. Therefore, when rotating the operation wire 40, it is possible to prevent the guide wire 43 from interfering with the operation wire 40 or the flexible sheath 25. In addition, the guide wire 43 is inserted through the through-hole 55, thereby negating the need for a space for providing a lumen, through which the guide wire 43 passes, in the flexible sheath 25. Therefore, in addition to the above effect of preventing the interference problem, the brush 45 can be quickly and appropriately operated depending on various situations while maintaining the flexible sheath 25 in a thin and soft state.

In addition, the operation wire 40 and the head chip 60 can be coupled with each other by using a simple structure which includes the recessed part 54, the coupling member 62, and the cylindrical fixed part 63, so that the operation wire 40 can be reliably rotated.

### Second embodiment

A second embodiment of the present invention will explained below.

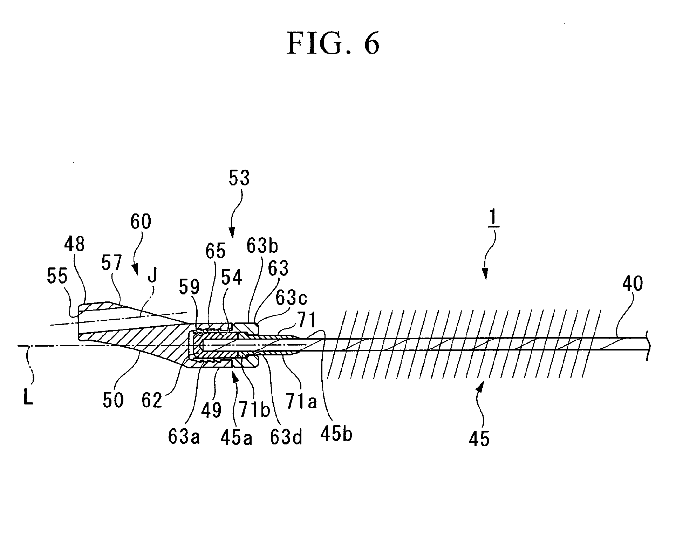

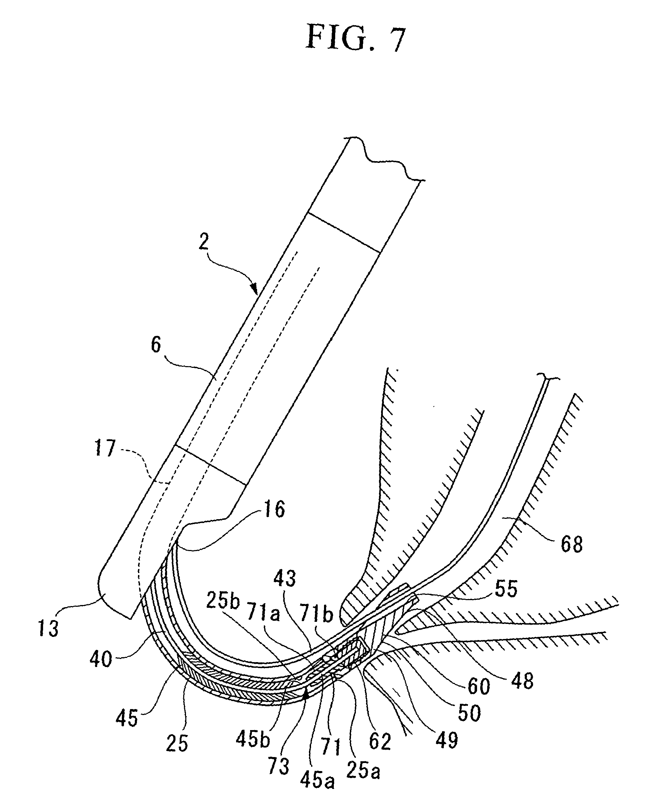

Figs. 6 and 7 show the second embodiment.

In Figs. 6 and 7, structural elements identical to those in Figs. 1 to 5 are given identical reference numerals, and explanations thereof are omitted.

Although the present embodiment has the same basic structure as the first embodiment, it also has the distinctive features as explained below.

In the guide wire treatment tool 1 of the present embodiment, as shown in Fig. 6, a cylindrical spacer 71 (i.e., a spacer member) is provided around the outer face 45b of the head part 45a in the brush 45. The cylindrical spacer 71 has a substantially cylindrical form, which extends along the axis L, and the head part 45a of the brush 45 is inserted through the opening of the cylinder. The cylindrical spacer 71 includes a spacer main body 71a, and an outward flange part 71 b which is formed at the head of main body 71 a and extends outward in the radial direction, where the main body 71a and the flange part 71b are integrally formed. The inward flange part 63 c is arranged in the recessed part 54, and the outward flange part 71 b contacts the inward flange part 63c, thereby preventing the cylindrical spacer 71 from becoming disconnected from the recessed part 54. In addition, the spacer main body 71 a extends toward the base end of the operation wire 40 via the opening 63d.

When the operation wire 40 is pulled so as to position the head chip 60 close to the head 25a of the flexible sheath 25, the spacer main body 71 a is inserted into the head 25a of the flexible sheath 25, as shown in Fig. 7. In this state, a space 73 between the outer face 45b of the head part 45a of the brush 45 and the inner face 25b of the flexible sheath 25 is substantially filled with the spacer main body 71 a along the entire circumference (in Fig. 7, the gap between the spacer main body 71 a and the inner face 25b of the flexible sheath 25 is enlarged for easier understanding).

In the above structure, when the head chip 60 is positioned close to the head 25a of the flexible sheath 25, the space 73 is substantially filled with the spacer main body 71 a. Therefore, if the head part 45a of the brush 45 tries to move in the flexible sheath 25 toward a direction which intersects the axis L, movement of the operation wire 40 is restricted by the spacer main body 71 a, thereby also restricting movement of the head chip 60 in the same direction.

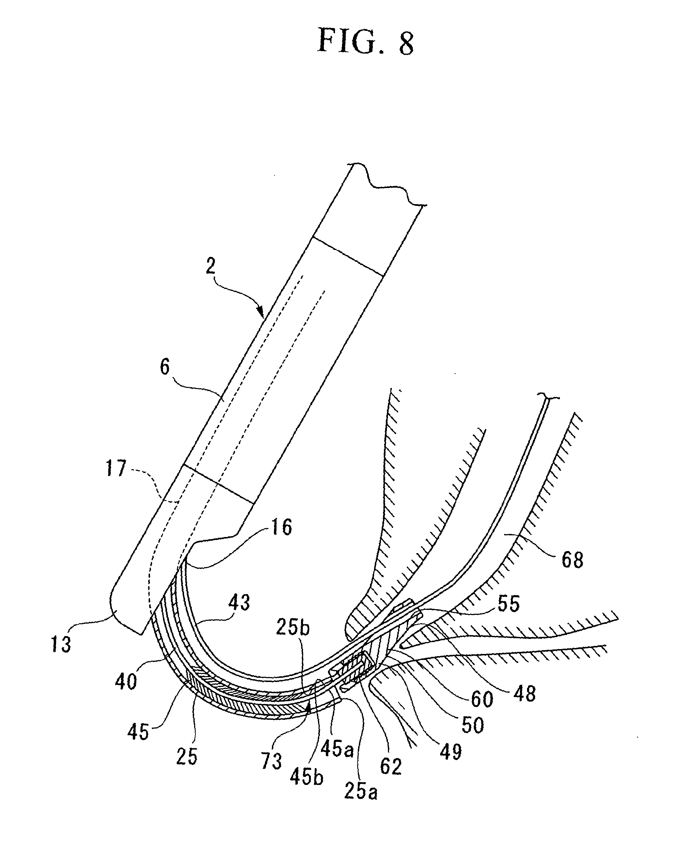

If the cylindrical spacer 71 is not installed, as shown in Fig. 8, the head chip 60 moves with respect to the flexible sheath 25 in a direction which intersects the axis L, so that the head chip 60 may be offset from the flexible sheath 25. When such an offset occurs, it may be difficult to insert the flexible sheath 25 into the bile duct 68 or to send the flexible sheath 25 along the bile duct 68.

As described above, in the guide wire treatment tool 1 of the present embodiment, when the head chip 60 is positioned close to the head 25a of the flexible sheath 25, movement of the head chip 60 is restricted, thereby preventing an offset between the head chip 60 and the flexible sheath 25. Therefore, it is possible to quickly and appropriately insert and send the flexible sheath 25.

In addition, the space 73 is filled with the spacer main body 71 a along the entire circumference thereof, thereby reliably restricting the movement of the head chip 60.

The above condition that the space 73 is filled with the spacer main body 71 a along the entire circumference is not absolute, and at least a part of the space 73 may be filled. However, of course, it is more preferable to fill the space along the entire circumference.

### Third embodiment

Next, a third embodiment will be explained.

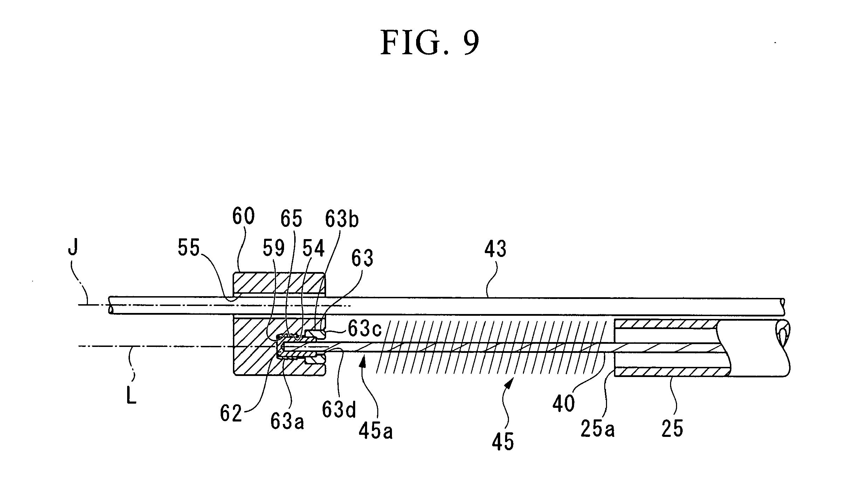

Fig. 9 shows the third embodiment.

In Fig. 9, structural elements identical to those in Figs. 1 to 5 are given identical reference numerals, and explanations thereof are omitted.

Although the present embodiment has the same basic structure as the first embodiment, it also has the distinctive features as explained below.

In the present embodiment, the longitudinal section of the head chip 60 has a substantially rectangular form. That is, the central axis J and the axis L are parallel to each other, and there is no rear-end cut part 57, which is provided in the previous embodiments.

Therefore, it is possible to prevent the guide wire 43 from interfering with the operation wire 40 or the flexible sheath 25, by using a simple structure.

### Fourth embodiment

Next, a fourth embodiment of the present invention will be explained.

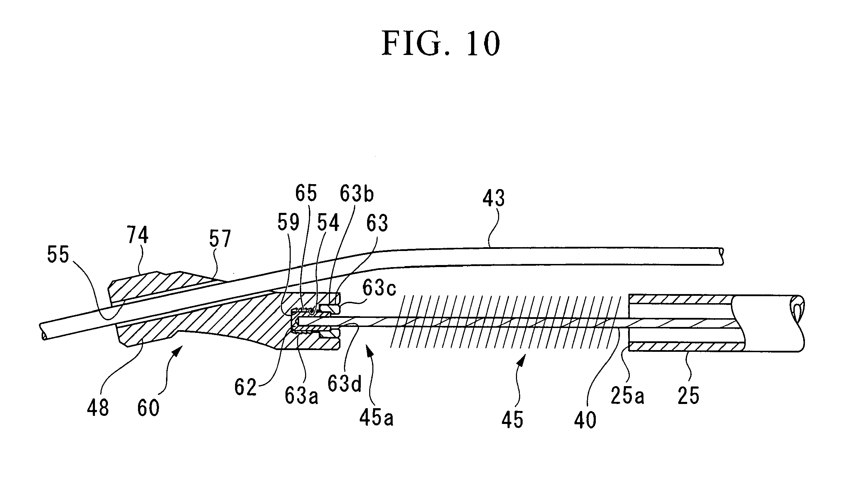

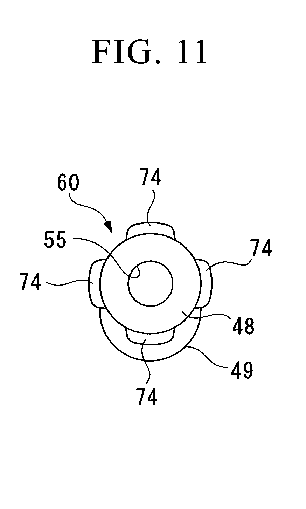

Figs. 10 and 11 show the fourth embodiment

In Figs. 10 and 11, structural elements identical to those in Figs. 1 to 5 are given identical reference numerals, and explanations thereof are omitted.

In the present embodiment, the head chip 60 has a rotation preventing device. That is, on the outer-peripheral face of the chip head part 48, protruding portions 74 are provided, each of which protrudes outward in a radial direction. The head of each protruding portion 74 is chamfered so as to have a substantially arc form. The protruding portions 74 extend along the central axis J, and are arranged at regular intervals along the circumferential direction (see Fig. 11).

In the above structure, when sending the operation wire 40 into a coelom, the head chip 60 smoothly moves forward without being obstructed by the protruding parts 74 which extend along the central axis J. On the other hand, when rotating the operation wire 40, a force for rotating the head chip 60 in the same direction may be applied to the head chip 60 (although such rotation is essentially prevented by rotation of the coupling member 62 in the recessed part 54, as described above), such a force is supported by the protruding parts 74. That is, the protruding parts 74 function as a rotation stopper, and thus as the rotation preventing device of the head chip 60.

Accordingly, when rotating the operation wire 40, it is possible to reliably rotate only the operation wire 40 without rotating the head chip 60.

### Fifth embodiment

Next, a fifth embodiment of the present invention will be explained.

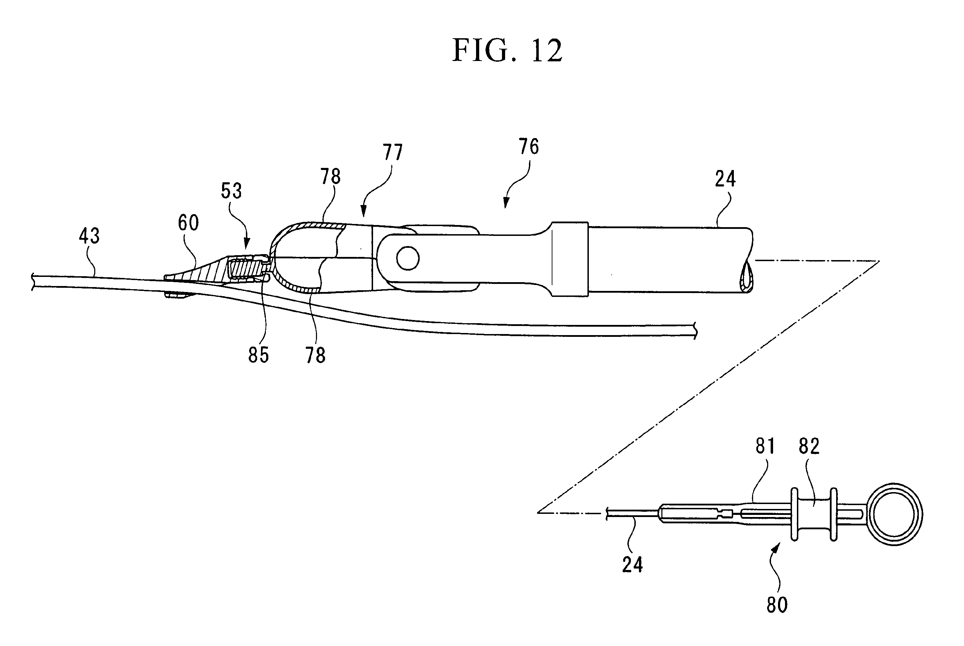

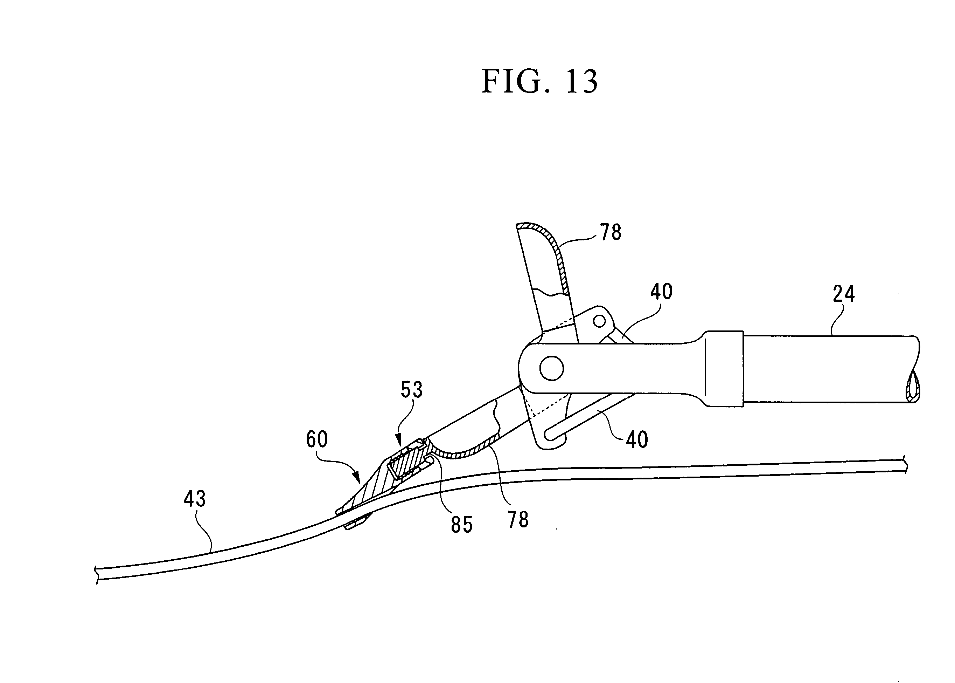

Figs. 12 and 13 show the fifth embodiment.

In the present embodiment, the head chip 60 is provided at the head of a pair of biopsy forceps 76. As shown in Fig. 12, in the forceps 76, a cup part 77 (i.e., a treatment part) for collecting tissue fragments is provided on the head of the sheath part 24. The cup part 77 has a pair of cup pieces 78, each of which extends along the axis of the sheath part 24, and has a bowl-form on the head thereof. On the other hand, on the base end of the sheath part 24, a forceps operating part 80 is provided, which has an operation main body 81 extending along the axis of the sheath part 24, and a slider 82, which is supported by the operation main body 81 in a slidable manner. The slider 82 and the base end of the pair of cup pieces 78 are coupled with each other via the operation wire 40. Therefore, as shown in Fig. 13, the cup pieces 78 are opened or closed by moving the slider 82 forward or backward.

A protruding part 85 is provided on the head of one cup piece 78, and the head chip 60 is provided similar to the above embodiments, via the protruding part 85.

In the above structure, the cup part 77 is sent to the narrowed part 69, similar to the above-described process. Then the slider 82 is operated so as to open the cup pieces 78, and the biopsy forceps 76 is pushed so as to push tissues against the inner faces of the cup pieces 78. Then, the cup pieces 78 are closed so as to collect tissue fragments. In the above process, in order to collect tissue fragments from a more appropriate area, the biopsy forceps 76 may be rotated so as to change the position of the cup part 77, depending on necessity.

The cup part 77 is made of heavy metal and thus hard, and also has an enlarged head so as to provide a space for collecting tissue fragments. Therefore, it may be difficult to insert the cup part into the bile duct 68 or the narrowed part 69. In the biopsy forceps 76 of the present embodiment, the head chip 60 is provided on the head of the cup part 77. Therefore, the biopsy forceps 76 can be guided to a target part along the guide wire 43, thereby easily inserting the cup part 77 into the bile duct 68 or the narrowed part 69. In addition, even when the biopsy forceps 76 is rotated, the head chip 60 is not moved. Therefore, similar to the above embodiments, interference with the guide wire 43 is also prevented.

### Sixth embodiment

Next, a sixth embodiment of the present invention will be explained.

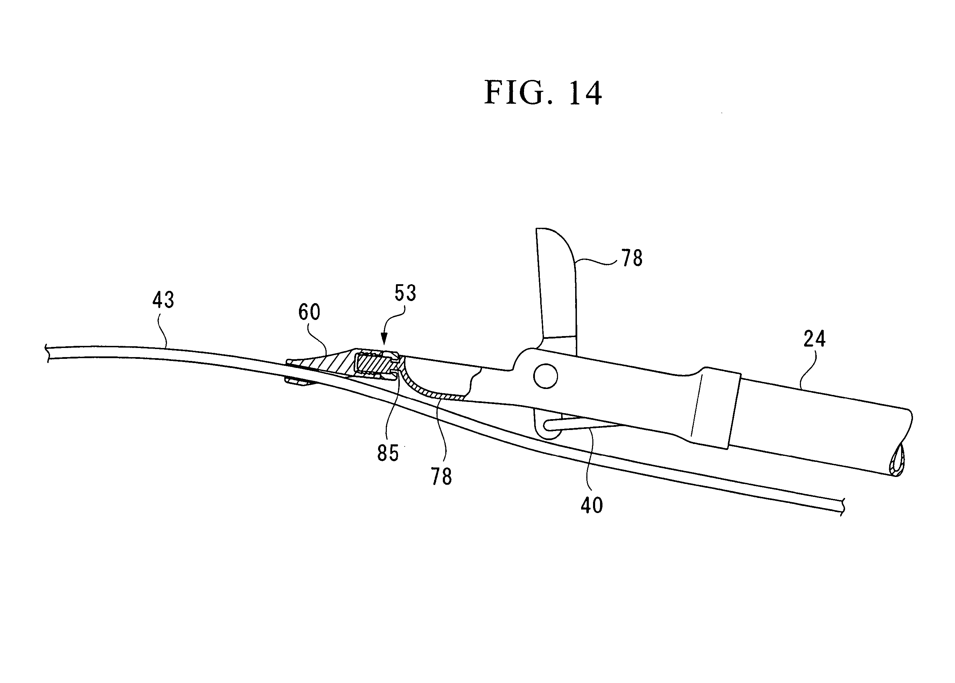

Fig. 14 shows the sixth embodiment.

In the present embodiment, the head of the operation wire 40 is connected to only one of the cup pieces 78, which are similar to those in the fifth embodiment. Therefore, the one cup piece moves when the cup pieces are opened. A protruding part 85 is provided on the head of the other cup piece 78, so as to provide the head chip 60 on the head of the protruding part 85.

Accordingly, the outer diameter when the cup pieces 78 are relatively opened can be reduced, thereby improving operability of the cup part 77 in the narrowed part 69.

In the first to sixth embodiments, the brush 45 is provided as the treatment part. However, this is not a limiting condition, and can be modified depending on the kind of treatment. For example, a basket or snare may be used instead.

The technical range is not limited to the above-described embodiments, and various modifications are possible without departing from the scope of the present invention.

## Claims

1. A guide wire treatment tool comprising:
an operation wire having a treatment part on the head thereof; and
a sheath through which the operation wire is slidably inserted,
wherein a head chip having a through-hole, through which a guide wire is inserted, is provided on a head part of the treatment part, and the treatment part and the head chip are coupled via a coupling part so as to be relatively rotatable around an axis which extends along the length of the operation wire.

2. The guide wire treatment tool in accordance with claim 1, wherein:
in the head chip, a recessed part is provided on an end thereof, wherein said end faces the treatment part;
the coupling part includes:
a coupling member, which is provided on the head part of the treatment part, and
a flange part, which extends inwardly in a radial direction and has an opening whose diameter is smaller than the outer diameter of the coupling member; and
the head part of the treatment part is rotatably held in the recessed part via the coupling member which is engaged with the flange part.

3. The guide wire treatment tool in accordance with claim 1, further comprising:
a spacer member, with which at least a part of a space between the inner face of the sheath and the outer face of the head part in the treatment part is filled when the head chip is arranged on the head of the sheath.

4. The guide wire treatment tool in accordance with claim 2, further comprising:
a spacer member, with which at least a part of a space between the inner face of the sheath and the outer face of the head part in the treatment part is filled when the head chip is arranged on the head of the sheath.
